# EUROPEAN PATENT SPECIFICATION

(11) **EP 0 836 443 B1**
(45) Date of publication and mention of the grant of the patent: **27.11.2002**
(21) Application number: 96917822.7
(22) Date of filing: 23.05.1996
(51) Int. Cl.: A61F 2/00

(54) **FEMALE URINARY INCONTINENCE DEVICE**
HARNINKONTINENZVORRICHTUNG FÜR FRAUEN
DISPOSITIF POUR L'INCONTINENCE URINAIRE FEMININE

(30) Priority: 07.06.1995 US 476092; 02.11.1995 US 556766; 26.12.1995 US 578031
(43) Date of publication of application: 22.04.1998
(73) Proprietor: Nebl,Inc., Worcester, MA 01609 (US)
(72) Inventor: BOGOJAVLENSKY, Sergei, Harvard, MA 01451 (US)
(74) Representative: Pratt, David Martin
(86) International application number: US9607517
(87) International publication number: WO96039989

(56) References cited:
- WO-A-90/08561
- DE-A- 3 633 824
- FR-A- 1 223 353
- GB-A- 1 467 144
- GB-A- 2 193 438
- US-A- 3 958 564

## Description

### Field of the Invention

The invention relates generally to a device for alleviating female urinary incontinence and more specifically to a device which constricts the meatus urinarius to alleviate female urinary incontinence.

### Background of the Invention:

Female urinary incontinence is a substantial problem throughout the world and can result from a variety of physical or mental dysfunctions. Neurogenic bladder dysfunction, trauma to the urethra or bladder neck, and injuries sustained during childbirth can all cause urinary incontinence in women. As well as being a medical problem, the condition can be socially embarrassing to women afflicted with the problem.

A variety of devices have been suggested to alleviate female urinary incontinence. Many of the suggested medical devices have internal components such as catheters, balloons and pessaries which pass into either the urethra or vagina and must remain positioned within the user's body when the device is in use. Several disadvantages exist with these internal devices. The internal components may be a source of irritation to the body or result in infection or other unwanted body reactions. Moreover, such devices as are known can be uncomfortable to wear, inconvenient to use, and expensive.

Therefore, it is important to provide a device that alleviates female urinary incontinence, does not create a high risk of body infection, can be comfortably worn, is easily applied and removed, and is inexpensive to provide.

The present invention provides the aforementioned desirable characteristics while avoiding the undesirable characteristics of prior art devices.

The present invention provides a device for alleviating female urinary incontinence comprising:
a resilient and at least partially-deformable device body having a hand gripping portion;
the device body defining a chamber therewithin sized to allow for reciprocal resilient deformation of the device body to provide a vacuum therein to hold the device on a user's body and close the meatus of the user's body.
the vacuum producing portion having a cylindrical shape with a substantially vertical cylindrical side wall centred around a central axis of the device and a rounded outer end wall;
the device body defining an encircling flange having a body-contacting surface to act as a sealing surface with the user's body;
the device acting to close the meatus of the user, thereby preventing urine leakage from the user's body.

### Brief Description of the Drawings

This invention is pointed out with particularity in the appended claims. The above and further advantages of this invention may be better understood by referring to the following description taken in conjunction with the accompanying drawings, in which:
Fig. 1 is a perspective view of an embodiment of a female urinary incontinence device having a circular body defining a chamber;
Fig. 2 is a cross-sectional view of the device of Fig. 1 taken through line 2-2' of Fig. 1;
Fig. 3 is a bottom view of the device of Fig. 1; and
Fig. 4 is a cross-sectional view taken through line 2-2' of Fig. 1 of the device of Fig. 1 when in place on the body of the user.

### Detailed Description of the Invention

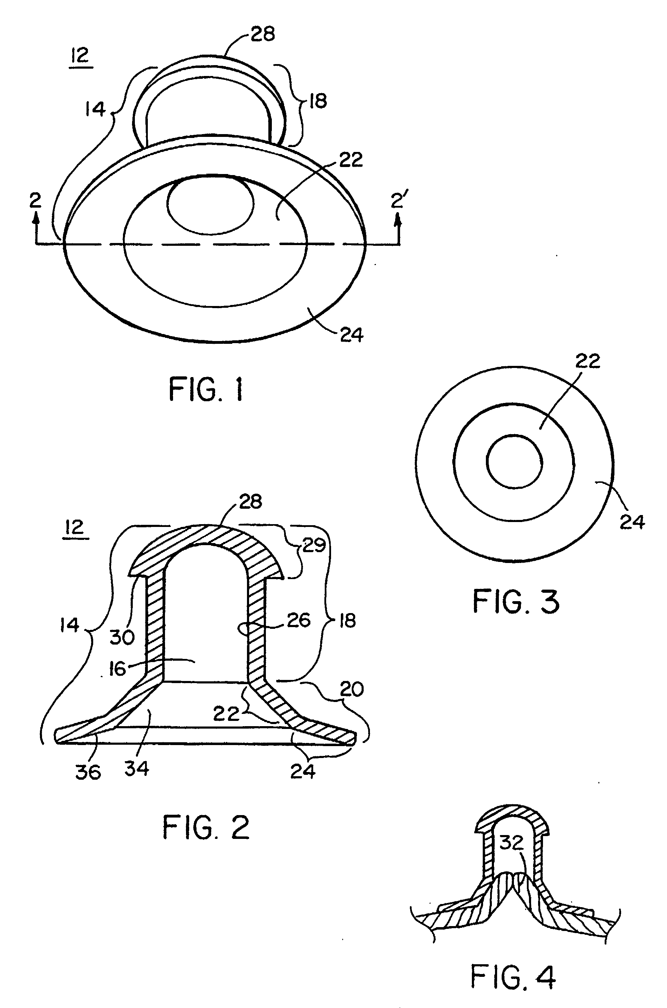

In broad overview and referring to Figs. 1-3, an embodiment of a female urinary incontinence device 12 of the invention, as shown in perspective view, cross-sectional view and bottom view in the respective Figures, includes a device body 14. Device body 14 defines a chamber 16 and has a vacuum producing portion 18 and a body contacting portion 20. The body contacting portion 20 comprises an intermediate frustoconical portion 22 and an outer encircling flange 24. The chamber 16 extends from the top of the device to the tip of the flange 24.

Figs. 1-3 show a vacuum producing portion 18 having a circular shape with a substantially vertical cylindrical sidewall 26 centered around the central axis of the device, a rounded outer endwall 28, and a finger gripping portion 29 with a finger gripping ledge 30.

In the preferred embodiment, device 12 is integrally formed by conventional molding. Also, in the preferred embodiment, device 12 is composed of an FDA approved material. While device 12 is typically a unitary part formed out of one piece of material, it may be formed out of multiple pieces of materials joined together. The vacuum producing portion 18 must be at least partially compressible in order to produce at least a partial vacuum in the chamber 16 sufficient to seal the device 12 to the user's body by a differential in air pressure between the air within the chamber 16 and the atmospheric air pressure. Production of the vacuum will be discussed in more detail below.

The thickness of the device body 14 is designed such that cylindrical sidewall 26 is thicker and more resistant to deformation or collapse by atmospheric pressure than is the flange 24 which tapers in thickness from the intermediate frustoconical portion 22 to the edge of the device body 14. In this embodiment both sidewall 26 and flange 24 can be formed of an FDA approved medical material. When the device 12 is in use, the difference in wall thickness prevents the device 12 from collapsing on itself, yet still allows the flange 24 to move towards the user's body.

Fig. 4 shows the urinary incontinence device 12 of Figs. 1-3 in use. As shown in Fig. 4, the flange 24 can be deformed towards or closely contact the user's body at the planar area of the user's body surrounding the meatus urinarius or urethra orifice. In one embodiment, the device body 14 can draw a portion of the orifice of the urethra 32 into contact with the flange 24 and intermediate frustoconical portion 22. Drawing a portion of the orifice of the urethra 32 into contact with the device body 14 acts to close the user's meatus to urine flow, to maintain the device 12 in position on the user's body, and to form a good seal between the user's body and the device 12 at flange 24.

In a preferred embodiment, when the device 12 is in use, the user's meatus is closed by a gentle compression of the area surrounding the meatus. This compression forms a closure which is maintained in position by the vacuum produced in the vacuum producing portion 18. Any structure that externally closes the meatus to urine flow, yet allows for comfort in use, can provide the advantages of this invention. These advantages can be obtained by the device 12 being applied solely externally of the user's body without any part of the device body 14 entering the user's body.

Figs. 1 and 2 show a finger gripping ledge 30 attached to the outer endwall 28. However, it is not necessary for device 12 to comprise finger gripping ledge 30. The finger gripping ledge 30 is important for placement particularly in older patients. Any configuration of device body 14 which is designed to allow the user to grip the device 12 by the user's fingers thereby allowing the user to position the device 12 on the user's body and to remove the device 12 from the user's body by the user's fingers is acceptable. In other embodiments, the finger gripping portion 29 can be simply the outer surface of the cylindrical outer sidewall 26 that surrounds the chamber 16. Thus, although Figs. 1-3 show device 12 having a cylindrical sidewall 26, rounded outer endwall 28, intermediate frustoconical portion 22, and outer encircling flange 24, the shape of device 12 can vary greatly.

The purpose of the intermediate frustoconical section 22 is to provide closure of the user's meatus. Typically, the angle of the body contacting surface 34 of the intermediate frustoconical portion 22 with the body contacting surface 36 of the outer encircling flange 24 is obtuse to enhance the closure of the meatus. The body contacting surface 34 of the intermediate frustoconical portion 22 defines the lower portion of chamber 16 and closes the meatus by pressing on the meatus.

Attached to the intermediate frustoconical portion 22 is the outer encircling flange 24 discussed above. In the preferred embodiment shown in Figs. 1-3, the outer encircling flange 24 has a body contacting surface 36 which forms a continuous ring about the meatus or opening of the user's urethra. However, other portions of the device 12, such as the vacuum producing portion 18, can be square, round, oblong, bulbous, or any shape desired. Outer endwall 28 may be flat rather than rounded. In all embodiments the chamber 16 has adequate volume to allow a vacuum to be formed within the chamber 16 sufficient to hold the device 12 onto the user's body.

The device 12 may come in many different sizes. However, consistent with normal anatomy of females in the United States, in the preferred embodiment, the body contacting portion 20 has an outer diameter preferably in the range of 2.3 to 3.4 centimeters with approximately 3.4 centimeters being preferred. The range of 2.3 to 3.4 centimeters in outer diameter is important for proper positioning of the device on the user's body and for maintaining the device 12 in place on the user's body. In the embodiments where the flange 24 has an encircling shape such as oval, square, oblong or triangular, the flange 24 can have a maximum width of approximately 3.4 centimeters. The outer encircling flange 24 has an inner diameter preferably in the range of 1.0 centimeter to 2.5 centimeters with approximately 1.5 centimeters being preferred. The device 12 has a height preferably in the range of 1.0 to 3.0 centimeters with approximately 2.0 centimeters being preferred. The height of device 12 can vary greatly, but by maintaining the height of device 12 between approximately 1.0 and 3.0 centimeters, the user can wear the device without discomfort and position the device easily. The height is preferably no more than approximately 3.0 centimeters to allow ease of use. In addition, when the height is maintained between approximately 1.0 and 3.0 centimeters, once the device is positioned on the user, it is covered by the vaginal labia and is resistant to dislodging by garments worn by the user.

In the preferred embodiment, the device 12 is integrally formed of elastomeric material.

Figs. 1-3 show an embodiment of the incontinence device 12 that is symmetrical about a central axis. However, the device 12 need not be symmetrical in all embodiments.

Fig. 4 shows the placement of the female urinary incontinence device 12 of Figs. 1-3 when in use. The user positions the flange 24 so that it lies substantially just within the user's labia on a substantially planar area surrounding the meatus. This spacing helps the user to locate and place the device 12 in position. When in position, the device 12 pulls the mucosa about the meatus into direct contact with the body contacting surfaces 34 and 36 of the intermediate frustoconical portion 22 and encircling flange 24 respectively. The body contacting surfaces 34 and 36 press against the mucosa surrounding the meatus to close the meatus and prevent urine outflow. Positioning the mucosa below the flange 24 aids in centering and maintaining the device 12 in position on the user's body.

Using a female urinary incontinence device 12 made of a resilient and partially deformable material is accomplished according to the following method. The user inwardly deforms the device body 14, applies the device body 14 over the orifice of the urethra and releases the device body 14 to allow the device body 14 to expand toward its original shape as shown in Fig. 2. As the device body 14 expands toward its original shape, a vacuum will be created within the inner chamber 16. The vacuum causes the outside atmospheric pressure to push against the flange 24 and intermediate frustoconical portion 22 and maintain the device 12 in good sealing engagement with the user's body. The air pressure difference compresses the mucosa or tissue immediately surrounding the meatus and forms a seal between the user's body and the device 12 at surfaces 34 and 36. The difference in pressure between the inside of the device 12 and the atmosphere can vary greatly. The difference in pressure is controlled in part by the amount the user depresses the chamber 16 before allowing the device body 14 to resiliently return toward its normal configuration as shown in Fig. 2. In some cases, the device body 14 does not fully return to its normal configuration after being compressed and applied to the user. However, in all cases, at least a partial vacuum remains inside the chamber 16. As well as containing a vacuum, the interior chamber 16 can act as a reservoir and store leakage that may occur while the device 12 is in place. In most embodiments of the device 12 leakage does not normally occur.

The difference in air pressure between the inside of the device 12 and the atmosphere is difficult to ascertain. In the preferred embodiment, since the device body's walls are resiliently deformable, the difference in pressure is created by the depression of the device body 14 and the expansion of the device body 14 towards its original shape after being applied to the user's body. The difference in air pressure may vary between applications even when the same device is used because the difference in air pressure depends on how the device is applied and how much the user depresses the device body 14. Surprisingly, it has been found that even with small devices designed and applied following the method of this invention, a sufficient difference in air pressure is created to maintain the device in position on the user's body and avoid urine flow.

Thus, a female user can alleviate urinary incontinence, such as stress incontinence, by applying the device 12 over the urethral orifice using the labia spacing to help position the device. To apply the device 12, prior to contacting the device body 14 with the user's body, the user resiliently depresses the device body 14 at the finger gripping portion 29. Once the device body 14 is depressed, the user brings the encircling flange 24 into contact with the mucosa surrounding the orifice of the urethra. With the device body 14 the user then applies slight pressure on the mucosa surrounding the orifice of the urethra. The user then releases the device body 14 causing a vacuum to be produced within the device. The vacuum provides the difference in air pressure which causes the atmosphere to press on the outside of the flange 24 and frustoconical portion 22. This pressure maintains the device 12 in place on the user's body and closes the user's meatus as shown in Fig. 4. When desired, the user can release the vacuum and easily remove the device 12 to allow voiding. The user may release the vacuum before removing the device 12 by slightly depressing the finger gripping portion 29. In some cases, the user can merely pull the device 12 off of the mucosa. The device 12 is comfortable to wear, can be easily applied by a majority of patients and has been found to prevent urinary leakage and thus alleviate urinary incontinence including stress incontinence in women.

In other embodiments, the device 12 of this invention can be sterilized prior to use to reduce the risk of infection or irritation to the user's skin. As the device is external to the user's body when in use and does not have any component passing within the urethra, the device does not require sterilization prior to use.

It has been found that devices of the type described above can be used for long periods of time and maintain in contact with and sealed to the user's skin for periods of 2 to 6 hours or more in some cases.

Having described preferred embodiments of the invention, it will now become apparent to one of skill in the art that other embodiments incorporating the concepts may be used. The particular materials, integral nature, and geometric configuration of the devices of this invention can vary greatly. In all embodiments, a difference in air pressure between the device's chamber 16 and the atmosphere is essential to provide a seal between the device and the user's body. The seal acts in conjunction with a Mechanical closure of the meatus to alleviate urinary incontinence. The seal formed between the flange 24, the intermediate frustoconical portion 22 and the user's body by the difference in air pressure is sufficiently strong to withstand and to prevent urinary flow out of the device over long periods of time at urinary pressures normally encountered at the urethral orifice.

## Claims

1. A device (12) for alleviating female urinary incontinence comprising:
a resilient and at least partially-deformable device body (14) having a hand gripping portion (29, 30);
the device body defining a chamber (16) therewithin sized to allow for reciprocal resilient deformation of the device body to provide a vacuum therein to hold the device on a user's body and close the meatus of the user's body.
the vacuum producing portion having a cylindrical shape with a substantially vertical cylindrical side wall (26) centred around a central axis of the device and a rounded outer end wall (28);
the device body defining an encircling flange (24) having a body-contacting surface (36) to act as a sealing surface with the user's body;
the device acting to close the meatus of the user, thereby preventing urine leakage from the user's body.

2. A device as claimed in claim 1, wherein the encircling flange (24) has an outer diameter of between substantially 2.3 and substantially 3.4 centimetres.

3. A device as claimed in claim 2, wherein the encircling flange (24) has an outer diameter of substantially 3 centimetres.

4. A device as claimed in any one of claims 1 to 3, wherein the central axis defined by the chamber (16) passes from the top of the device body (14) to the bottom of the device body, with the bottom being defined by the encircling flange (24), the top to bottom having a height of substantially 2 centimetres.

5. A device as claimed in any one of claims 1 to 4, wherein the device (12) is integrally formed of a resilient material which allows ease of application to the user's body by deforming the chamber (16) of the device body (14), applying the device to the user's body about the orifice of the urethra, and releasing the deforming pressure to define an air pressure difference between the chamber and the atmosphere sufficient to seal the encircling flange (24) to the user's body, and to prevent liquid flow therethrough at normal pressures encountered in urinary fluids expressed by the body,
the device further defining a meatus-constricting surface (34) to close the meatus when the device is applied with the pressure difference acting to position the device.

## Patentansprüche

1. Vorrichtung (12) zum Lindern weiblicher Harninkontinenz, umfassend:
einen elastischen und zumindest teilweise verformbaren Vorrichtungskörper (14) mit einem Greifabschnitt (29, 39);
wobei der Vorrichtungskörper darin eine Kammer (16) definiert, welche derart bemessen ist, dass eine reziproke elastische Verformung des Vorrichtungskörpers gewährleistet ist, um darin ein Vakuum zum Halten der Vorrichtung auf dem Körper einer Benutzerin und zum Schließen des Meatus des Körpers der Benutzerin zu erzeugen;
wobei der ein Vakuum erzeugende Abschnitt eine zylindrische Form mit einer im wesentlichen vertikalen zylindrischen Seitenwand (26), welche um eine Mittelachse der Vorrichtung mittig angeordnet ist, und einer gerundeten äußeren Stirnwand (28) aufweist;
wobei der Vorrichtungskörper einen umgebenden Flansch (24) definiert, welcher eine Körperkontaktfläche (36) aufweist, die als Dichtungsfläche mit dem Körper der Benutzerin dient;
wobei die Vorrichtung derart wirkt, dass diese den Meatus der Benutzerin schließt, wodurch ein Austreten von Urin aus dem Körper der Benutzerin verhindert wird.

2. Vorrichtung nach Anspruch 1, wobei der umgebende Flansch (24) einen Außendurchmesser von zwischen im wesentlichen 2,3 und im wesentlichen 3,4 Zentimetern aufweist.

3. Vorrichtung nach Anspruch 2, wobei der umgebende Flansch (24) einen Außendurchmesser von im wesentlichen 3 Zentimetern aufweist.

4. Vorrichtung nach einem der Ansprüche 1 bis 3, wobei die durch die Kammer (16) definierte Mittelachse ausgehend vom oberen Abschnitt des Vorrichtungskörpers (14) hin zum unteren Abschnitt des Vorrichtungskörpers verläuft, wobei der untere Abschnitt durch den umgebenden Flansch (24) definiert ist und die Höhe vom oberen Abschnitt bis zum unteren Abschnitt im wesentlichen 2 Zentimeter beträgt.

5. Vorrichtung nach einem der Ansprüche 1 bis 4, wobei die Vorrichtung (12) einstückig aus einem elastischen Material hergestellt ist, welches eine einfache Anwendung auf den Körper der Benutzerin durch Verformen der Kammer (16) des Vorrichtungskörpers (14), Anwenden der Vorrichtung auf den Körper der Benutzerin um die Öffnung der Harnröhre und Aufheben des Verformungsdrucks, um eine Luftdruckdifferenz zwischen der Kammer und der Atmosphäre zu definieren, welche ausreicht, den umgebenden Flansch (24) zum Körper der Benutzerin zu dichten und einen Flüssigkeitsstrom dadurch hindurch bei normalen Drücken zu verhindern, welche in vom Körper abgegebenen Urinflüssigkeiten vorhanden sind, ermöglicht,
wobei die Vorrichtung ferner eine Meatus-verengende Fläche (34) definiert, um den Meatus zu schließen, wenn die Vorrichtung angewendet wird, wobei die Druckdifferenz zum Anordnen der Vorrichtung dient.

## Revendications

1. Dispositif (12) pour soulager l'incontinence urinaire féminine comprenant :
. un corps (14) élastique et au moins partiellement déformable présentant une partie de préhension manuelle (29, 30) ;
. le corps délimitant intérieurement une chambre (16) dimensionnée pour permettre la déformation élastique réciproque du corps afin d'établir un vide dans celui-ci pour maintenir le dispositif sur le corps d'une utilisatrice et fermer son méat ;
. la partie produisant le vide affectant une forme cylindrique avec une paroi latérale verticale sensiblement cylindrique (26) centrée autour d'un axe central du dispositif et une paroi d'extrémité externe arrondie (28) ;
. le corps définissant une bordure plate de ceinture (24) présentant une surface de contact corporel (36) destinée à agir en tant que surface d'étanchéité avec le corps de l'utilisatrice ;
. le dispositif agissant pour fermer le méat de l'utilisatrice, empêchant ainsi des fuites d'urine provenant du corps de l'utilisatrice.

2. Dispositif selon la revendication 1, **caractérisé en ce que** la bordure plate de ceinture (24) présente un diamètre externe compris sensiblement entre 2,3 et 3,4 centimètres.

3. Dispositif selon la revendication 2, **caractérisé en ce que** la bordure plate de ceinture (24) présente un diamètre sensiblement égal à 3 centimètres.

4. Dispositif selon l'une quelconque des revendications 1 à 3, **caractérisé en ce que** l'axe central défini par la chambre (16) passe du sommet du corps (14) vers le fond du corps, le fond étant défini par la bordure plate de ceinture (24), la distance entre le sommet et le fond étant sensiblement égale à 2 centimètres.

5. Dispositif selon l'une quelconque des revendications 1 à 3, **caractérisé en ce que** le dispositif (12) est formé d'une seule pièce en une matière élastique qui permet une application aisée sur le corps de l'utilisatrice par déformation de la chambre (16) du corps (14), application du dispositif sur le corps de l'utilisatrice au niveau de l'orifice de l'urètre, et relâchement de la pression de déformation pour établir une différence de pression d'air entre la chambre et l'atmosphère suffisante pour ajuster la bordure plate de ceinture (24) de manière étanche au corps de l'utilisatrice, et pour empêcher l'écoulement de liquide à travers celui-ci à des pressions normales pour des fluides urinaires évacués par le corps, le dispositif définissant en outre une surface de constriction du méat (34) pour fermer le méat lorsque le dispositif est appliqué avec la différence de pression agissant pour positionner le dispositif.
